# EUROPÄISCHE PATENTSCHRIFT

(11) **EP 0 845 476 B1**
(45) Veröffentlichungstag und Bekanntmachung des Hinweises auf die Patenterteilung: **14.03.2001**
(21) Anmeldenummer: 97121976.1
(22) Anmeldetag: 17.06.1992
(51) Int. Cl.: C07H 21/00, C12Q 1/68, C12N 9/00

(54) **Synthetische katalytische Oligonukleotide**
Synthetic catalytic oligonucleotides
Oligonucléotides synthétiques catalytiques

(30) Priorität: 20.06.1991 DE 4120406; 15.05.1992 DE 4216134
(43) Veröffentlichungstag der Anmeldung: 03.06.1998
(62) Teilanmeldung aus: 92110298.4
(73) Patentinhaber: EUROPÄISCHES LABORATORIUM FÜR MOLEKULARBIOLOGIE (EMBL), D-69117 Heidelberg (DE)
(72) Erfinder: Sproat, Brian, Dr., 37139 Adelebsen (DE); Lamond, Angus, Dr., Newport-on-Tay, Fife DD6 8NN Scotland (GB); Paolella, Giovanni, Dr., 69251 Gaiberg (DE)
(74) Vertreter: Viering, Hans-Martin, Dipl.-Ing.

(56) Entgegenhaltungen:
- EP-A- 0 360 257
- EP-A- 0 427 073
- EP-A- 0 427 074
- US-A- 4 469 863
- A.M.IRIBARREN ET AL.: "2'-O-Alkyl Oligoribonucleotides as Antisense Probes." PROCEEDINGS OF THE NATIONAL ACADEMY OF SCIENCES OF THE USA, Bd. 87, Nr. 19, 1990, WASHINGTON US, Seiten 7747-7751, XP002060667
- A.HAMPEL ET AL.: "RNA Catalytic Properties of the Minimum (-)sTRSV Sequence." BIOCHEMISTRY, Bd. 28, Nr. 12, 1989, EASTON PA US, Seiten 4929-4933, XP002060668
- B.S.SPROAT ET AL.: "New Synthetic Routes to Synthons Suitable for 2'-O-Allyloligoribonucleotide Assembly." NUCLEIC ACIDS RESEARCH, Bd. 19, Nr. 4, 1991, Seiten 733-738, XP002061363
- B.S.SPROAT ET AL.: "New Synthetic Routes to Protected Purine 2'-O-Methylriboside-3'-O-Phosphoramidites Using a Novel Alkylation Procedure." NUCLEIC ACIDS RESEARCH, Bd. 18, Nr. 1, 1990, ARLINGTON VIRGINIA US, Seiten 41-49, XP002060669
- HAMPEL A ET AL: "'HAIRPIN' CATALYTIC RNA MODEL: EVIDENCE FOR HELICES AND SEQUENCE REQUIREMENT FOR SUBSTRATE RNA" NUCLEIC ACIDS RESEARCH, Bd. 18, Nr. 2, 1990, ARLINGTON VIRGINIA US, Seiten 299-304, XP002043044
- C.J.HUTCHINS AT AL.: "Self-Cleavage of Plus and Minus RNA Transcripts of Avocado Sunblotch Viroid." NUCLEIC ACIDS RESEARCH , Bd. 14, Nr. 9, 1986, ARLINGTON VIRGINIA US, Seiten 3627-3640, XP002060670

## Beschreibung

Die vorliegende Erfindung betrifft synthetische katalytische Oligonukleotidstrukturen, die zur Spaltung einer Nukleinsäure-Zielsequenz geeignet sind und modifizierte Nukleotide enthalten. Die Erfindung betrifft weiterhin ein Verfahren zur Spaltung einer Nukleinsäure-Zielsequenz unter Verwendung von synthetischen katalytischen modifizierten Oligonukleotidstrukturen.

Nach Entdeckung der von RNA vermittelten Katalyse wurden verschiedene Versuche der Inaktivierung von spezifischen RNA-Molekülen in vitro und in vivo unternommen. Von besonderem Interesse hat sich die Entdeckung erwiesen, daß Hammerkopf-Ribozyme zur Entwicklung eines Vielzweckenzyms dienen können, welches zur Erkennung und Spaltung einer gegebenen spezifischen RNA zumindestens in vitro in der Lage ist (Haseloff und Gerlach (1988)). Viele interessante Einsatzmöglichkeiten für derartige Ribozyme beruhen auf ihrer Fähigkeit, eine spezifische RNA innerhalb eines gegebenen Gemisches effizient zu erkennen und zu spalten. Die Einsatzmöglichkeiten für RNA-Enzyme reichen von der Entwicklung von RNA-Restriktionsenzymen bis zur spezifischen Inaktivierung von Genen in der Zelle. Ein besonderes biomedizinisches Interesse beruht auf der Tatsache, daß viele Krankheiten einschließlich vieler Tumorformen in Korrelation zur Expression spezifischer Gene stehen. Die Inaktivierung solcher Gene durch Spaltung der zugehörigen mRNA würde einen möglichen Weg zur Kontrolle und schließlichen Heilung solcher Krankheiten darstellen. Ferner besteht ein großes Bedürfnis zur Entwicklung von antiviral wirksamen Arzneimitteln, wobei die RNA-Enzyme möglicherweise ein solches Mittel sein könnten, da die virale Expression selektiv durch Spaltung der viralen RNA-Moleküle blockiert werden kann.

Bisherige Versuche zur Expression von Ribozymen in der Zelle durch Transfektion der Zelle mit dem entsprechenden Gen hat sich als nicht sehr wirksam erwiesen, da zur Inaktivierung der spezifischen RNA eine sehr hohe Expression erforderlich war. Wahrscheinlich ist auch die direkte Verabreichung von RNA-Molekülen aufgrund der Sensitivität von RNA gegenüber Abbau durch RNAsen und ihren Wechselwirkungen mit Proteinen unmöglich.

Es bestand daher ein sehr großes Bedürfnis, RNA-Enzyme zu entwickeln, bei denen die Nachteile des Standes der Technik mindestens teilweise beseitigt sind.

Die der Erfindung zugrundeliegende Aufgabe wird dadurch gelöst, daß man anstelle eines RNA-Moleküls einen neuen Nukleinsäuretyp verwendet, der auf artifiziellen Nukleotiden mit einem 2'-Alkoxy-Substituenten beruht. Solche Moleküle sind erheblich stabiler als native RNA-Moleküle, da sie weder durch RNAsen noch durch DNAsen gespalten werden und auch weniger Wechselwirkungen mit RNA- oder DNA-bindenden Proteinen eingehen (Iribarren et al., Proc.Natl.Acad.Sci. USA 87 (1990), 7747-7751). Derartige Moleküle versprechen eine grössere Wirksamkeit in der zellulären Umgebung als entsprechende native RNA-Moleküle.

Solche Moleküle sind aus dem Stand der Technik bekannt. Zum Beispiel beschreiben Ts'o und Miller in US-A-4 469 863 nichtionische Alkyl- und Arylphosphonatnukleinsäuren sowie Verfahren zu deren Herstellung. In Nuc. Acids Res. 18 (1990) 41-49 beschreiben Sproat et al. neue synthetischen Wege zu Purin-2'-O-Methylribosid-3'-O-Phosphoramiditen mittels eines neuen Alkylationsverfahrens. Schließlich beschreiben Sproat et al. in Nuc. Acids Res. 19 (1991) 733-738 neue synthetischen Wege zu Synthonen, die zur Konstruktion von 2'-O-Allyloligoribonukleotiden geeignet sind.

Diese modifizierten Nukleinsäuren sind jedoch nornalerweise nicht als Katalysatoren, wie sie von Hampel et al. in EP-A-0 360 257 ("RNA catalyst for cleaving specific RNA sequences") beschrieben sind wirksam, es wurde jedoch überraschenderweise festgestellt, daß ihre Aktivität erhalten bleibt, wenn man eine sehr geringe Anzahl von Hydroxylresten an spezifischen Positionen des Moleküls einbaut. Dabei bleiben überraschenderweise auch ihre charakteristischen Eigenschaften, insbesondere die Stabilität und die verringerte Wechselwirkung mit Proteinen erhalten.

Ein Gegenstand der vorliegenden Erfindung ist daher eine synthetische katalytische Oligonukleotidstruktur, die zur Spaltung einer Nukleinsäure-Zielsequenz geeignet ist und Nukleotiden mit der allgemeinen Strukturformel (I) enthält: 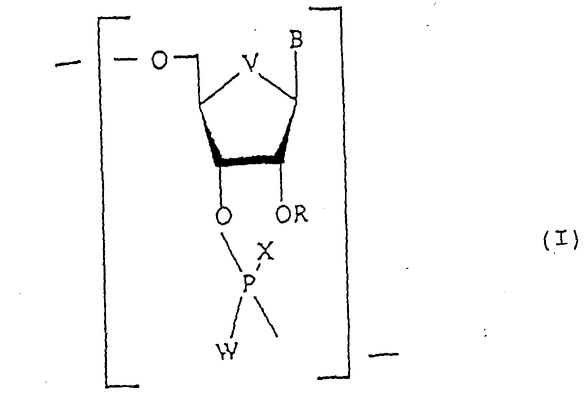 worin B eine Nukleosidbase darstellt, die insbesondere aus der Gruppe, bestehend aus Adenin-9-yl (A), Cytosin-1-yl (C), Guanin-9-yl (G), Uracil-1-yl (U), Uracil-5-yl (ψ), Hypoxanthin-9-yl (I), Thymin-1-yl (T) und 2-Aminoadenin-9-yl ausgewählt ist,
V bei jedem Nukleotid unabhängig eine O- oder eine CH₂-Gruppe ist,
X und W jeweils in einem Nukleotid gleich oder verschieden sein können und unabhängig voneinander O-, S-, NH₂-, Alkyl-oder Alkoxygruppen mit 1 bis 10, vorzugsweise mit 1 bis 4 Kohlenstoffatomen sind,
R Wasserstoff oder eine geradkettige oder verzweigte, gegebenenfalls mit Halogen-, Cyano-, Isocyano-, Nitro-, Amino-, Carboxyl-, Hydroxyl- oder/und Mercaptogruppen substituierte Alkyl-, Alkenyl- oder Alkinylgruppe mit 1 bis 10 Kohlenstoffatomen ist, welche dadurch gekennzeichnet ist, daß bei mindestens einem der Nukleotide der Rest R in Formel (I) von Wasserstoff verschieden ist, daß innerhalb des katalytisch aktiven Zentrums Nukleotide exisitieren, in denen R Wasserstoff ist, daß das katalytisch aktive Zentrum der Oligonukleotidstruktur eine Hammerkopf- oder Haarnadelstruktur aufweist, und daß die Oligonukleotidstruktur Bereiche enthält, die für die Hybridisierung mit der Nukleinsäure-Zielsequenz verantwortlich sind.

B kann eine beliebige Purin- oder Pyrimidinnukleosidbase darstellen. Beispiele für geeignete Purinnukleosidbasen sind etwa Adenin-9-yl, Guanin-9-yl, Hypoxanthin-9-yl und 2-Amino-adenin-9-yl. Beispiele für Pyrimidinnukleosidbasen sind etwa Cytosin-1-yl, Uracil-1-yl, Uracil-5-yl und Thymin-1-yl.

V, X und W sind jeweils O-Gruppen (wobei in diesem Fall ein O-Atom über eine Doppelbindung an ein Phosphor gebunden wäre und das andere über eine Einfachbindung gebunden wäre und eine negative Ladung aufweisen würde).

R ist Wasserstoff oder eine geradkettige oder verzweigte, gegebenenfalls mit Halogen (Fluor, Chlor, Brom, Jod), Cyano-, Isocyano-, Nitro-, Amino-, Carboxyl-, Hydroxyl- oder/und Mercaptogruppen substituierte Alkyl-, Alkenyl- oder Alkinylgruppen mit 1 bis 10 Kohlenstoffatomen. Die von Wasserstoff verschiedenen Reste R enthalten vorzugsweise 1 bis 6 Kohlenstoffatome. Beispiele für bevorzugte Reste R sind Methyl-, Ethyl-, Propyl-, Allyl-, Dimethylallyl-, Butyl- oder Cyanomethylreste. Besonders bevorzugt ist R ein Alkenylrest mit 1 bis 4 Kohlenstoffatomen, z.B. ein Allylrest.

Eine erfindungsgemäße katalytische Oligonukleotidstruktur ist dadurch gekennzeichnet, daß bei mindestens einem der Nukleotide der Rest R in Formel (I) von Wasserstoff verschieden ist. Dabei ist es bevorzugt, wenn die Oligonukleotidstruktur eine möglichst geringe Anzahl von Nukleotiden enthält, in denen R Wasserstoff bedeutet. Derartige Oligonukleotide weisen eine hohe Resistenz gegenüber Nukleasen auf und zeigen geringere Wechselwirkungen mit Nukleinsäure-bindenden Proteinen. Andererseits darf nicht bei allen Nukleotiden der Rest R von Wasserstoff verschieden sein, da ansonsten das Oligonukleotid keine katalytische Aktivität mehr aufweist. Insbesondere innerhalb des katalytisch aktiven Zentrums des erfindungsgemäßen Oligonukleotids existieren einzelne Nukleotide, in denen der Rest R ein Wasserstoffatom darstellt.

Das katalytisch aktive Zentrum der erfindungsgemäßen Oligonukleotidstruktur weist vorzugsweise eine Hammerkopf- oder eine Haarnadelstruktur auf.

Katalytische Haarnadelstrukturen sind z.B. in den Arbeiten von Tritz und Hampel (Biochemistry 28 (1989), 4929) und Hampel et al., Nucleic Acids Res. 18 (1990), 299-304 beschrieben. Eine derartige Haarnadelstruktur enthält 4 Helices, von denen 2 zwischen dem Substrat und der katalytischen RNA ausgebildet sind. Im folgenden ist ein Beispiel für ein katalytisches Zentrum in Form einer RNA-Haarnadelstruktur angegeben, die aus dem Tobacco-Ringspot-Virus stammt:

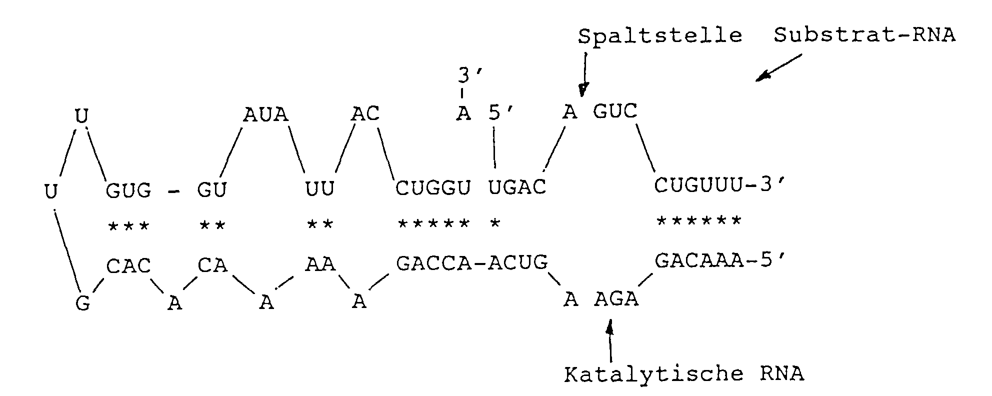

Das aktive Zentrum einer katalytischen RNA kann auch eine sogenannte Hammerkopf-Struktur besitzen (Hotchins et al., Nucleic Acids Res. 14 (1986), 3627; Kiese und Symons in: Viroids and viroid-like pathogens, J.S. Semancik, Herausgeber (CRC-Press, Bocaratan, Florida (1987), S. 1-47). Das katalytische Zentrum der Hammerkopfstruktur enthält 3 Stämme und kann durch benachbarte Sequenzbereiche der RNA oder aber auch durch Bereiche gebildet werden, die durch viele Nukleotide voneinander getrennt sind.

Gegenstand der vorliegenden Erfindung ist daher eine Hammerkopf-Oligonukleotidstruktur mit der allgemeinen Strukturformel (II): 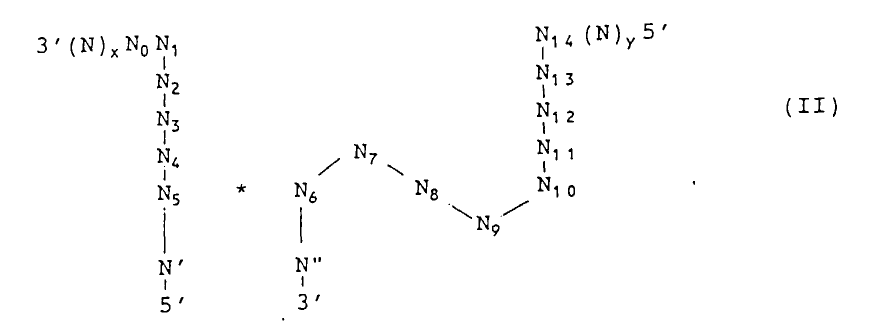 worin N jeweils ein Nukleotid gemäß der allgemeinen Strukturformel (I) darstellt,
x und y gleich oder verschieden sein können und x ≥ 1 und y ≥ 2 ist,
N₅ und N₆ zueinander jeweils komplementäre Nukleotide sind und * eine Basenpaarung darstellt,
N' und N" entweder zwei Nukleotidsequenzen darstellen, die mindestens teilweise zueinander komplementäre Nukleotide enthalten, so daß eine stabile Basenpaarung zwischen den beiden Nukleotidsequenzen ermöglicht wird, oder
N' und N" zusammen eine einzige Nukleotidsequenz darstellen, wobei mindestens ein Teil der Sequenz durch Basenpaarung zwischen komplementären Nukleotiden einen doppelsträngigen Stamm bilden kann,
und worin gegebenenfalls ein oder mehrere zusätzliche Nukleotide N nach N₇ oder/und N₉ insertiert werden können; welche dadurch gekennzeichnet ist, daß bei mindestens einem der Nukleotide N₂, N₃, N₅, N₆, N₇, N₉, N₁₀ N₁₂ N₁₃ und N₁₄ in Formel (II) der Rest R in Formel (I) von Wasserstoff verschieden ist.

Der Bereich N₁ bis N₁₄ enthält das katalytische Zentrum der Oligonukleotidstruktur (II). Die mit (N)ₓ und (N)_{y} bezeichneten Nukleotide befinden sich außerhalb des aktiven Zentrums und enthalten Bereiche, die für die Hybridisierung mit einer spezifischen Nukleinsäure-Zielsequenz verantwortlich sind. Die Länge dieser Bereiche ist so, daß x ≥ 1 und y ≥ 2 sein muß. Vorzugsweise sind x und y ≤ 20, größere Werte für x oder/und y bringen keine spezifischen Vorteile, erschweren aber die Synthese des Oligonukleotids. Die Oligonukleotidstruktur II kann ein entweder zusammenhängendes Molekül sein oder aus zwei verschiedenen Molekülen bestehen, d.h. N' und N" stellen entweder zusammen eine einzige Nukleotidsequenz oder zwei verschiedene Nukleotidsequenzen dar. Für die erfindungsgemäße Struktur ist wesentlich, daß die Nukleotidsequenzen N' und N" mindestens teilweise zueinander komplementäre Bereiche enthalten, die eine stabile Basenpaarung zwischen beiden Nukleotidsequenzen ermöglichen. Unter dem Begriff einer stabilen Basenpaarung ist dabei zu verstehen, daß die Oligonukleotidstruktur unter physiologischen Bedingungen bei Raumtemperatur und vorzugsweise bei Temperaturen bis zu 40°C als doppelsträngiger Strang vorliegt.

Die erfindungsgemäße Oligonukleotidstruktur zeichnet sich aus, daß bei mindestens einem, vorzugsweise bei mehreren der Nukleotide N₂, N₃, N₅, N₆, N₇, N₉, N₁₀, N₁₂, N₁₃ und N₁₄ der Rest R in Formel (I) von Wasserstoff verschieden ist. Bei den Nukleotiden N₁, N₄, N₈ und N₁₁ sind hingegen die Reste R in Formel (I) vorzugweise Wasserstoff. Weiterhin ist bevorzugt, wenn die Nukleosidbase bei N₁ Adenin-1-yl oder 2-Aminoadenin-9-yl und bei den Nukleotiden N₄, N₈ und N₁₁ jeweils Guanin-1-yl (oder Hypoxanthin-9-yl) ist.

Ein besonders bevorzugter Gegenstand der vorliegenden Erfindung ist eine Oligonukleotidstruktur mit der allgemeinen Strukturformel (III): 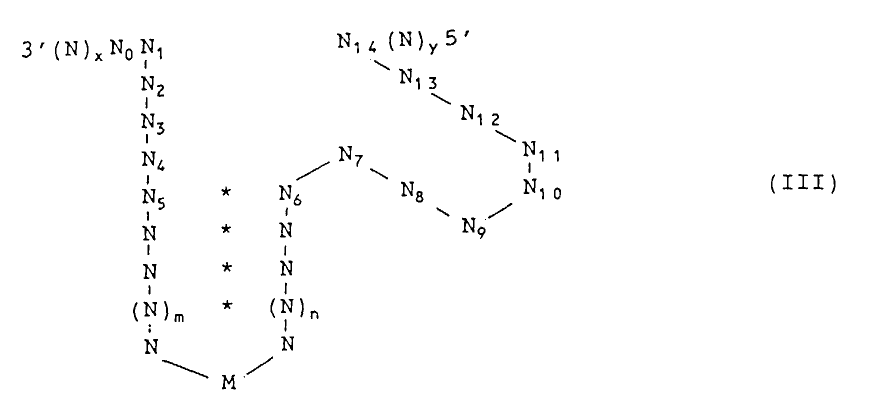 worin N, x und y wie in Anspruch 3 definiert sind,
M eine chemische Bindung darstellt oder eine Nukleotidsequenz (N)ₐ bedeutet, wobei a ≥ 1 ist, m und n gleich oder verschieden sind und gegebenenfalls ein oder mehrere zusätzliche Nukleotide nach N₇ oder/und N₉ insertiert werden können.

Die Reste R in Formel (III) bei den Nukleotiden N₁, N₄, N₈, N₁₀, N₁₁ und N₁₂ sind vorzugsweise Wasserstoff. Die Reste R in Formel (III) bei allen Nukleotiden außer N₁, N₄, N₈, N₁₀, N₁₁ und N₁₂ sind vorzugsweise von Wasserstoff verschieden.

Ein bevorzugtes konkretes Beispiel für ein erfindungsgemäßes Oligonukleotid mit einer Hammerkopfstruktur als katalytisches Zentrum besitzt eine Struktur gemäß Formel (II) oder (III) und ist dadurch gekennzeichnet, daß die Reste V, W und X in Formel (I) O-Gruppen sind und daß bei den Nukleotiden N₁ bis N₁₄ in Formel (I) oder (III) die Reste B und R die folgenden Bedeutungen besitzen:
N₁ : B = A und R = H,
N₂ : B = A und R = Allyl,
N₃ : B = A und R = Allyl,
N₄ : B = G und R = H,
N₅ : B = C und R = Allyl,
N₆ : B = G und R = Allyl,
N₇ : B = A und R = Allyl,
N₈ : B = G und R = H,
N₉ : B = U und R = Allyl,
N₁₀ : B = A und R = H,
N₁₁ : B = G und R = H,
N₁₂ : B = U und R = H,
N₁₃ : B = C und R = Allyl,
N₁₄ : B = U und R = Allyl.

Ein weiteres bevorzugtes konkretes Beispiel für ein Oligonukleotid mit einer Hammerkopfstruktur als katalytisches Zentrum ist dadurch gekennzeichnet, daß die Reste V, W und X in Formel (I) O-Gruppen sind mit der Ausnahme, daß die Verbindung zwischen N₁₁ und N₁₂ eine Phosphorthioatgruppe (X = O und W = S) ist, und daß bei den Nukleotiden N₁ bis N₁₄ in Formel (I) oder (III) die Reste B und R die obigen Bedeutungen besitzen.

Ein weiteres beispielhaftes Oligonukleotid ist dadurch gekennzeichnet, daß die Reste V, W und X in Formel (I) O-Gruppen sind und daß bei den Nukleotiden N₁ bis N₁₄ in Formel (I) oder (III) die Reste B die obige Bedeutung besitzen und daß für N₁, N₄, N₈, N₁₀, N₁₁ und N₁₂ R = H, für N₉ R = 3,3-Dimethylallyl und für N₂, N₃, N₅, N₆, N₇, N₁₃ und N₁₄ R = Allyl ist. Ein weiteres Oligonukleotid unterscheidet sich von der letztgenannten Struktur nur dadurch, daß für N₃ R = 3,3-Dimethylallyl und für N₉ R = Allyl ist.

In noch einer weiteren Struktur ist für N₁, N₄, N₈, N₁₀, N₁₁ und N₁₂ R = H, für N₂, N₃, N₇, N₉ und N₁₃ R = Cyanomethyl und für N₅, N₆ und N₁₄ R = Allyl, wobei die Reste B die oben angegebenen konkreten Bedeutungen besitzen.

Weiterhin kann es auch bevorzugt sein, daß bei den erfindungsgemäßen Oligonukleotidstrukturen einer oder mehrere der Reste R = Butyl sind, um die Aufnahme der katalytischen Strukturen durch die Zelle zur verbessern. Ein konkretes Beispiel hierfür ist ein Oligonukleotid, bei dem für N₁, N₄, N₈, N_{10,} N₁₁ und N₁₂ R = H, für N₅ und N₆ R = Butyl und für N₂, N₃, N₇, N₉, N₁₃ und N₁₄ R = Allyl ist, wobei gegebenenfalls für einen, mehrere oder alle der Reste N, die in Formel (III) in basengepaarter Form (durch einen Stern gekennzeichnet) vorliegen, R ebenfalls Butyl sein kann.

Zur zusätzlichen Stabilisierung der erfindungsgemäßen Oligonukleotidstrukturen können sich an ihrem freien 3'-Ende bzw. an ihren freien 3'-Enden 3'-Deoxyribonukleotide oder/und 3'-O-Alkylribonukleotide befinden. Auf diese Weise wird das erfindungsgemäße Oligonukleotid vor einem 3'-Exonukleaseabbau geschützt.

Die erfindungsgemäßen Oligonukleotide können weiterhin zur Stabilisierung ihrer räumlichen Konfiguration Nukleotide enthalten, deren Nukleosidbasen durch ein Quervernetzungsmittel modifiziert sind. Ein Beispiel für ein derartiges Quervernetzungsmittel ist Psoralen oder ein Psoralenderivat. Auf diese Weise können doppelsträngige Oligonukleotidstrukturen durch kovalente Quervernetzung modifizeirt werden. Die Herstellung von Nukleotiden, die mit einem Quervernetzungsmittel modifiziert sind, ist detailliert in der DE-A 39 28 900 offenbart.

Ferner kann die erfindungsgemäße Oligonukleotidstruktur mit einer prostethischen Gruppe verknüpft sein, um die Aufnahme in der Zelle oder/und die spezifische zelluläre Lokalisierung der Oligonukleotidstruktur zu verbessern. Beispiele für derartige prostethische Gruppen sind Polyaminosäuren (z.B. Polylysin), Lipide, Hormone oder Peptide. Die Verknüpfung dieser prostethischen Gruppen erfolgt üblicherweise über freie 5'-bzw. 3'-Enden der erfindungsgemäßen Oligonukleotidstruktur, wobei diese Verknüpfung entweder direkt oder über einen Linker erfolgen kann. Beispiele für Linker sind etwa am terminalen 5'-Phosphat vorliegende Diester mit Amino- oder Phosphatfunktion oder mit Merkaptoalkoxygruppen.

Die Synthese der erfindungsgemäßen Oligonukleotidstrukturen erfolgt auf an sich bekannte Weise aus Monomereinheiten. Derartige Monomereinheiten besitzen üblicherweise die allgemeine Formel (V) 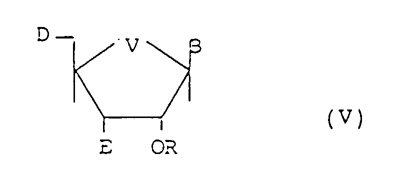 worin V, B und R wie in Formel (I) definiert sind und
D und E zur Ausbildung von 3'- bis 5'-Internukleotidbindungen fähige reaktive Gruppen bedeuten. Solche Gruppen sind dem Fachmann bekannt und z.B. in B. Sproat et al., Nucleic Acids Res. 18 (1990), 41-49 sowie zusammenfassend in E.L. Winnacker, Gene und Klone, VCH-Verlagsgesellschaft mbH, Weinheim (Deutschland) (1985), insbesondere Seiten 44-49 und in Froehler und Matteucci, Tetrahedron Let. (1986), S. 469-472 beschrieben. Mit reaktiven Mononukleotiden der Formel (V) lassen sich auf bekannte Weise, insbesondere an einer festen Phase die erfindungsgemäßen Oligonukleotidstrukturen herstellen.

Ein weiterer Gegenstand der vorliegenden Erfindung ist ein Verfahren zur Spaltung einer Nukleinsäure-Zielsequenz unter Verwendung einer synthetischen katalytischen Oligonukleotidstruktur gemäß vorliegender Erfindung. Diese Nukleinsäure-Zielsequenz kann dabei entweder ein Teil der synthetischen katalytischen Oligonukleotidstruktur selbst sein, oder es kann sich bei dieser Nukleinsäure-Zielsequenz um ein von der synthetischen katalytischen Oligonukleotidstruktur verschiedenes Molekül handeln.

Verwendet man zur Spaltung einer Nukleinsäure-Zielsequenz ein erfindungsgemäßes Oligonukleotid mit einer Hammerkopfstruktur der allgemeinen Formel (II), so wird üblicherweise eine Zwischenstufe mit der folgenden Struktur ausgebildet: 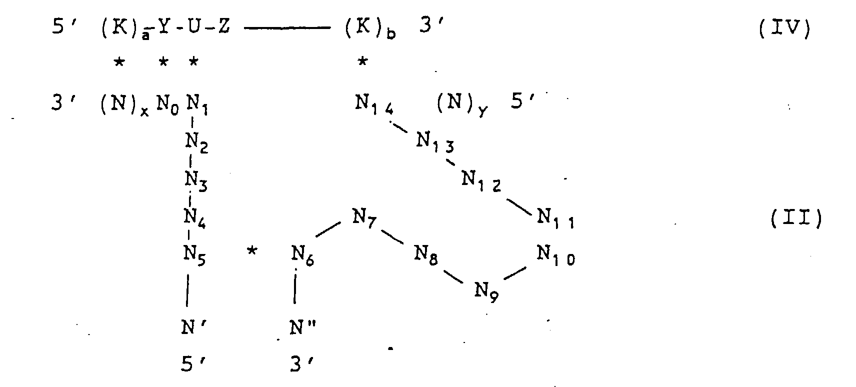 wobei die Symbole N, N', N", x, y und * wie in Anspruch 3 definiert sind,
und K, Y, U und Z Nukleotide der Nukleinsäure-Zielsequenz darstellen,
worin U Uridin ist,
Z ein nicht modifiziertes Ribonukleotid, ausgewählt aus der Gruppe, bestehend aus Adenosin, Cytidin oder Uridin ist,
K und Y beliebige Nukleotide sind,
a ≥ 1 und b ≥ 3 ist,
und die Spaltung der Nukleinsäure-Zielsequenz 3'-seitig der Nukleotidsequenz YUZ erfolgt, und wobei gegebenenfalls eine chemische Bindung zwischen dem 5'-Ende der Nukleinsäure-Zielsequenz (IV) und dem 3'-Ende des Oligonukleotids (II) bei (N)ₓ oder zwischen dem 3'-Ende der Nukleinsäure-Zielsequenz (IV) und dem 5'-Ende des Oligonukleotids (II) bei (N)_{y} existiert.

Das Nukleotid Y in der Nukleinsäure-Zielsequenz stellt vorzugsweise einen Guanosinrest dar und Z bedeutet vorzugsweise Adenosin oder Cytidin. Die Nukleinsäure-Zielsequenz kann ein beliebiges Oligo- oder Polynukleotid sein, vorausgesetzt daß Z ein nicht-modifiziertes Ribonukleotid ist. Der Rest der Zielsequenz kann beispielsweise DNA, 2'-O-Alkyl-RNA oder eine gemischte Struktur sein. Vorzugsweise ist die Nukleinsäure-Zielsequenz jedoch eine RNA. Die Spaltungsspezifität des erfindungsgemäßen Oligonukleotids für eine bestimmte Nukleinsäure-Zielsequenz kann dadurch erreicht werden, indem die Sequenz der Hybridisierungsarme der katalytischen Komponente (N₀(N)X bzw. (N)YN₁₄) so verändert werden, daß sie zu den Sequenzen komplementär sind, welche die Spaltstelle der gewünschten Zielsequenz flankieren.

Das erfindungsgemäße Verfahren kann sowohl innerhalb einer lebenden Zelle als auch in vitro durchgeführt werden. Vorzugsweise wird die Spaltung in Gegenwart eines divalenten Kations, insbesondere Mg²⁺ und bei einem pH-Wert von etwa 7 bis 9, besonders bevorzugt etwa 8 durchgeführt.

Ein weiterer Gegenstand der vorliegenden Erfindung ist die Verwendung einer erfindungsgemäßen Oligonukleotidstruktur zur katalytischen Spaltung einer Nukleinsäure-Zielsequenz, wobei die Nukleinsäure-Zielsequenz entweder ein Teil der katalytischen Oligonukleotidstruktur ist oder ein davon verschiedenes Molekül darstellt.

Ferner betrifft die Erfindung ein Arzneimittel, das als Wirkstoff eine erfindungsgemäße Oligonukleotidstruktur, gegebenenfalls zusammen mit üblichen pharmazeutischen Träger-, Hilfs-, Füll- oder/und Verdünnungsmitteln enthält. Die Erfindung betrifft auch ein Verfahren zur Herstellung eines Arzneimittels für die antivirale Therapie in Menschen, Tieren und Pflanzen, wobei man als Wirkstoff eine erfindungsgemäße Oligonukleotidstruktur verwendet. Ein Beispiel für eine derartige antivirale Therapie wäre die Bekämpfung von AIDS mit den erfindungsgemäßen Oligonukleotiden (siehe z.B. Sarver et al., Science 247 (1990), 1222-1225).

Ein weiterer Gegenstand der vorliegenden Erfindung ist auch ein diagnostisches Reagenz, das als Bestandteil eine erfindungsgemäße Oligonukleotidstruktur enthält sowie ein Verfahren zur Herstellung eines derartigen diagnostischen Reagenz. Dieses erfindungsgemäße diagnostische Reagenz kann beispielsweise für eine genetische Musterungsprozedur verwendet werden.

Die Erfindung soll weiterhin durch die folgenden Beispiele und die Sequenzprotokolle SEQ ID NO.1 und SEQ ID NO.2 verdeutlicht werden.

Es zeigen:
- SEQ ID NO.1:: die Nukleotidsequenz einer Substrat-RNA und
- SEQ ID NO.2:: die Nukleotidsequenz einer katalytisch aktiven Ribozym-RNA

### Beispiel 1

### Oligonukleotidsynthese, Deblockierung und Reinigung

Die Synthese von 2'-O-Methylribonukleosid-3'-O-phosphoramidit-Bausteinen erfolgte nach Sproat et al. (Nucleic Acids Res. 18 (1990), 41-49). 2'-O-Allylribonukleosid-3'-O-phosphoramidit-Bausteine wurden nach Sproat et al. (Nucleic Acids Res. 19 (1991), 733-738) synthetisiert.

2'-O-[1-(2-Fluorphenyl)-4-methoxypiperidin-4-yl]ribonukleosid-3'-O-phophoramidit-Bausteine wurden nach Beijer et al. (Nucleic Acids Res. 18 (1990), 5143-5151) synthetisiert.

Die Oligonukleotide wurden nach dem β-Cyanoethylphosphoramidit-Verfahren auf Controlled Pore Glass unter Verwendung eines modifizierten DNA-Synthesezyklus auf einen Applied Biosystems Synthetisierapparat zusammengesetzt. Anstelle von Tetrazol wurde 5-(4-Nitrophenyl)-1H-Tetrazol als Aktivator für den Kondensationsschritt verwendet, wobei die Reaktionsdauer für die Kondensation auf 12 Minuten erhöht wurde (vgl. Nucleic Acids Res. 18 (1990), 41-49 und 5141-5151).

Die Deblockierung und Reinigung wurde durchgeführt, indem ein Träger, an dem ein völlig blockiertes Oligonukleotid gebunden war, zunächst mit einer Lösung von 25 %igem wäßrigem Ammoniak in einem abgedichteten sterilen Gefäß für 10 Stunden bei 60°C behandelt wurde. Die abgekühlte Lösung wurde dann in einem sterilen Gefäß im Vakuum bis zur Trockene eingedampft. Das Oligonukleotid-Rohprodukt, welches noch eine 5'-terminale 5'-O-Dimethoxytrithyl-Schutzgruppe und einige 2'-O-Fpmp-Schutzgruppen enthält, wurde dann durch Reverse Phase HPLC auf einer µ-Bondapak C₁₈-Säule unter Verwendung eines Acetonitrilgradienten in wäßrigem 0,1 mol/l Triethylammoniumacetat pH 7,0 als Elutionsmittel gereinigt. Der Produktpeak wurde gesammelt und das Lösungsmittel im Vakuum entfernt. Der Rückstand wurde in 1 ml 10 %igem Glycerin in Wasser resuspendiert und die Lösung wurde 10 Minuten zentrifugiert. Der Überstand wurde auf eine G15 Sephadex-Säule (30 ml) gegeben, die mit sterilem destilliertem Wasser eluiert wurde. Das Leervolumen der Säule (etwa 9 ml) wurde verworfen und eine 3 ml-Fraktion wurde gesammelt, welche das teilweise blockierte Oligonukleotid enthielt. Dann wurde sterile wäßrige Salzsäure (270 µl, 0,1 mol/l) zugegeben, wodurch ein pH im Bereich von 2 bis 2,5 erhalten wurde. Die Lösung wurde 20 Stunden lang bei 20 bis 25°C gehalten, um die säurelabilen DMTr- und Fpmp-Schutzgruppen zu entfernen. Anschließend wurde die Lösung 10 Minuten bei 2000 Upm zentrifugiert. Der Überstand wurde durch Zugabe von 2 mol/l Tris Acetat (75 µl, pH 7,9) neutralisiert. Das reine vollständig entblockierte Oligonukleotid wurde aus der wäßrigen Lösung unter Verwendung von wiederholten Extraktionen mit 1-Butanol gewonnen, wie bei Cathala und Brunel (Nucleic Acids Res. 18 (1990), 201) beschrieben. Das Oligonukleotidpellet wurde getrocknet und in 100 µl Tris-Puffer (10 mmol/l, pH 7,5, 1 mmol/l EDTA) gelöst.

### Beispiel 2

### Herstellung und Markierung von Substrat-RNA

Chemisch synthetisierte Substratoligonukleotide wurden entweder mit γ-³²P-ATP und Kinase oder mit RNA-Ligase und ³²P-pCp gemäß den von den Herstellern der jeweiligen Enzyme vorgeschlagenen Standardprozeduren markiert.

Alternativ dazu wurde RNA auch durch Transkription mit SP6-RNA-Polymerase nach Linearisierung der jeweiligen Matrizen mit geeigneten Restriktionsenzymen synthetisiert. Die RNA-Moleküle wurden durch Einbau von α-³²P-UTP in das Transkript markiert. Die Bedingungen der Transkription waren wie folgt:
40 mmol/l Tris, pH 7,5
6 mmol/l MgCl₂
2 mmol/l Spermidin
10 mmol/l Dithiotreitol
500 mmol/l Nukleosidtriphosphate (A, C, G)
100 µmol/l UTP
10 µCi α-³² P-UTP
10 U/µl SP6-Polymerase
20 U/µl RNAse-Inhibitor (menschliche Plazenta)

Nach zweistündiger Inkubation bei 37°C wurde die als Matrize verwendete DNA mit RNAse-freier DNAse verdaut. RNA wurde durch Gelelektrophorese auf 6 %igen Polyacrylamidgelen (1:30 Acrylamid:Bisacrylamid) in Gegenwart von 7 mol/l Harnstoff aufgetrennt. RNA-Banden wurden durch Diffusion eluiert und RNA wurde durch Ethanolpräzipitation gewonnen. Die Aktivitätsbestimmung wurde in einer 10 µl Reaktionsmischung ausgeführt, welche Substrat RNA (10 nM bis 1 µM), Tris 50 mM pH 7,5, MgCl₂ 20 mM, 0,01 bis 10 pMol Ribozym enthält. Diese Mischung wurde bei 50°C 60 Minuten inkubiert. Die Spaltprodukte wurden auf 7 % Polyacrylamidgel in Gegenwart von 7 M Harnstoff aufgetrennt.

### Beispiel 3

Bestimmung der Aktivität unterschiedlicher, modifizierter, katalytischer Oligonukleotide.

Als Zielsequenz wurde ein 17 Nukleotide langes, chemisch synthetisiertes Oligoribonukleotid, basierend auf dem EDB Exon menschlicher Fibronektin-mRNA, verwendet. Die Sequenz (SEQ ID NO.1) war wie folgt:

5'- r[UACACAGUCACAGGGCU]

Das zur Spaltung dieser Sequenz verwendete Ribozym besaß die im folgenden dargestellte Nukleotidsequenz (SEQ ID NO.2):

5'- r[GCCCUGUCUGAUGAGUCCGUGAGGACGAAACUGUGU]

Diese Sequenz ist als E0 bezeichnet. Die unterstrichenen Bereiche bezeichnen die Nukleotide N₁₄ bis N₆ bzw. N₅ bis N₀ gemäß Formel (III). Weiterhin wurden die folgenden Analoga von E0 (mit einer identischen Basenzusammensetzung) synthetisiert:
E1: R = H für N₁, N₄ , N₈, N₁₀, N₁₁ und N₁₂; R = Allyl für alle anderen N.
E2: R = H für N₁, N₄ , N₈, N₁₀ und N₁₁ R = Allyl für alle anderen N.
E3: R = H für N₁, N₄, N₈, N₁₀, N₁₁ und N₁₂; die Verbindung zwischen N₁₁ und N₁₂ ist eine Phosphorthioatgruppe (d.h. X = O und W = S gemäß Formel (I)); R = Allyl für alle anderen N.
E4: R = H für N₁, N₄ , N₈, N₁₀, N₁₁ und N₁₂; R = 3,3-Dimethylallyl für N₉ und R = Allyl für alle anderen N.
E5: R = H für N₁, N₄, N₈, N₁₀, N₁₁ und N₁₂; R = 3,3-Dimethylallyl für N₃ und R = Allyl für alle anderen N.
E6: R = H für N₁, N₄, N₈, N₁₀, N₁₁ und N₁₂; R = Butyl für N₅ und N₆ sowie für die anderen 6 N (UCC und GGA), die einen basengepaarten Bereich bilden. Für alle anderen N ist R = Allyl.
E7: R = H für N₁, N₄, N₈, N₁₀, N₁₁ und N₁₂; R = Cyanomethyl für N₂, N₃, N₇, N₉ und N₁₃ ; R = Allyl für alle restlichen N.

Der Test zur Bestimmung der Spaltaktivität wurde in einem Reaktionsvolumen von 10 µl durchgeführt, das Substrat RNA (10 nmol/l bis 1 µmol/l), Tris 50 mmol/l pH 7,5, MgCl₂ 20 mmol/l und 0,01 bis 10 pmol Ribozym enthielt. Das Reaktionsgemisch wurde 60 Minuten lang bei 50°C inkubiert. Die Spaltprodukte wurden durch Polyacrylamid-Gelelektrophorese in Gegenwart von 7 mol/l Harnstoff aufgetrennt.

Die Reaktionskinetik wurde durch Messung der Menge an ³²P-radioaktiv markiertem Substrat bei einer Spaltungsdauer von 1,5, 10 und 15 Minuten bei 50°C bestimmt. Die Substratkonzentrationen waren wie folgt: 25, 50, 100, 250 nmol/l; die Ribozymkonzentration war zwischen 4 nmol/l und 20 nmol/l. Die Proben wurden vorgewärmt und die Reaktion wurde durch Zugabe von Ribozym gestartet. Nach der vorbestimmten Reaktionsdauer wurde die Reaktion durch Zugabe 2 Volumina 20 mmol/l EDTA gestoppt. Die Produkte wurden durch Polyacrylamid-Harnstoff-Gelelektrophorese aufgetrennt und mit dem Molecular Dynamics Phosphor Imager quantitativ analysiert.

In der folgenden Tabelle 1 sind die relative Aktivität und die RNase-Sensitivität der oben aufgelisteten katalytischen Oligonukleotide E0, E1, E2, E3, E4, E5, E6 und E7 angegeben.

**Tabelle 1**

| Katalytische Struktur | Relative Aktivität | RNase A Sensitifität |
|---|---|---|
| E0 | 1 | 1 |
| E1 | 0,2 | 0,01 |
| E2 | < 0,05 | stabil |
| E3 | 0,1 | 0,001 |
| E4 | ca. 0,2 | 0,01 |
| E5 | ca. 0,2 | 0,01 |
| E6 | ca. 0,2 | 0,01 |
| E7 | ca. 0,2 | 0,01 |

SEQ ID NO.1:
Nukleinsäure (RNA):
einzelsträngig
linear
Länge: 17 UACACAGUCA CAGGGCU 17 SEQ ID NO.2:
Nukleinsäure (RNA):
einzelsträngig
linear
Länge: 36

GCCCUGUCUG AUGAGUCCGU GAGGACGAAA CUGUGU 36

## Patentansprüche

1. Synthetische katalytische Oligonukleotidstruktur, die zur Spaltung einer Nukleinsäure-Zielsequenz geeignet ist und Nukleotide mit der allgemeinen Strukturformel (I) enthält: 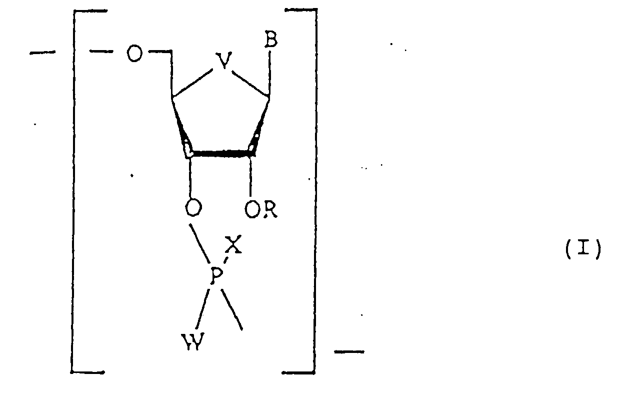 worin B eine Nukleosidbase darstellt, die insbesondere aus der Gruppe, bestehend aus Adenin-9-yl (A), Cytosin-1-yl (C), Guanin-9-yl (G), Uracil-1-yl (U), Uracil-5-yl (ψ), Hypoxanthin-9-yl (I), Thymin-1-yl (T) und 2-Amino-adenin-9-yl ausgewählt ist,
V bei jedem Nukleotid unabhängig eine O- oder eine CH₂-Gruppe ist,
X und W jeweils in einem Nukleotid gleich oder verschieden sein können und unabhängig voneinander O-, S-, NH₂-, Alkyl- oder Alkoxygruppen mit 1 bis 10, vorzugsweise mit 1 bis 4 Kohlenstoffatomen sind,
R Wasserstoff oder eine geradkettige oder verzweigte, gegebenenfalls mit Halogen-, Cyano-, Isocyano-, Nitro-, Amino-, Carboxyl-, Hydroxyl- oder/und Mercaptogruppen substituierte Alkyl-, Alkenyl- oder Alkinylgruppe mit 1 bis 10 Kohlenstoffatomen ist,
**dadurch gekennzeichnet,**
daß bei mindestens einem der Nukleotide der Rest R in Formel (I) von Wasserstoff verschieden ist, daß innerhalb des katalytisch aktiven Zentrums Nukleotide exisitieren, in denen R Wasserstoff ist, daß das katalytisch aktive Zentrum der Oligonukleotidstruktur eine Hammerkopf- oder Haarnadelstruktur aufweist, und daß die Oligonukleotidstruktur Bereiche enthält, die für die Hybridisierung mit der Nukleinsäure-Zielsequenz verantwortlich sind.

2. Oligonukleotidstruktur nach Anspruch 1,
**dadurch gekennzeichnet,**
daß die Reste V, W und X in Formel (I) O-Gruppen sind.

3. Oligonukleotidstruktur nach Anspruch 1 oder 2,
**dadurch gekennzeichnet,**
daß die von Wasserstoff verschiedenen Reste R in Formel (I) 1 bis 6 Kohlenstoffatome enthalten.

4. Oligonukleotidstruktur nach Anspruch 3,
**dadurch gekennzeichnet,**
daß die von Wasserstoff verschiedenen Reste R in Formel (I) aus der Gruppe, bestehend aus Methyl-, Ethyl-, Propyl-, Allyl-, Dimethylallyl-, Butyl- oder Cyanomethylresten ausgewählt sind.

5. Oligonukleotidstruktur nach einem der vorhergehenden Ansprüche,
**dadurch gekennzeichnet,**
daß sich an den freien 3'-Enden der Oligonukleotidstruktur 3'-Deoxyribonukleotide oder/und 3'-O-Alkylribonukleotide befinden.

6. Oligonukleotidesturktur nach einem der vorhergehenden Ansprüche,
**dadurch gekennzeichnet,**
daß sie weiterhin zur Stabilisierung ihrer räumlichen Konfiguration Nukleotide enthält, deren Nukleosidbasen durch ein Quervernetzungsmittel modifiziert sind.

7. Oligonukleotidstruktur nach Anspruch 6,
**dadurch gekennzeichnet,**
daß das Quervernetzungsmittel Psoralen oder ein Psoralenderivat ist.

8. Oligonukleotidstruktur nach einem der vorhergehenden Ansprüche,
**dadurch gekennzeichnet,**
daß sie mit einer prostethischen Gruppe, ausgewählt aus Polyaminosäuren, Lipiden, Hormonen oder Peptiden verknüpft ist, um die Aufnahme in der Zelle oder/und die spezifische zelluläre Lokalisierung der Oligonukleotidstruktur zu verbessern.

9. Verfahren zur Spaltung einer Nukleinsäure-Zielsequenz unter Verwendung einer synthetischen katalytischen Oligonukleotidstruktur nach einem der Ansprüche 1 bis 10.

10. Verfahren nach Anspruch 9,
**dadurch gekennzeichnet,**
daß die Nukleinsäure-Zielsequenz eine Ribonukleinsäure ist.

11. Verfahren nache Anspruch 9 oder 10,
**dadurch gekennzeichnet,**
daß man die Spaltung in Gegenwart eines divalenten Kations, insbesondere Mg2+ durchführt.

12. Verfahren nach einem der Ansprüche 9 bis 11,
**dadurch gekennzeichnet,**
daß man die Spaltung bei einem pH-Wert von etwa 7 bis 9 durchführt.

13. Verwendung einer Oligonukleotidstruktur nach einem der Ansprüche 1 bis 8 zur katalytischen Spaltung einer Nukleinsäure-Zielsequenz.

14. Arzneimittel,
**dadurch gekennzeichnet,**
daß es als Wirkstoff eine Oligonukleotidstruktur nach einem der Ansprüche 1 bis 8, gegebenenfalls zusammen mit üblichen pharmazeutischen Träger-, Hilfs-, Füll-oder/und Verdünnungsmitteln enthält.

15. Verwendung einer Oligonukleotidstruktur nach einem der Ansprüche 1 bis 8 zur Herstellung eines Arzneimittels für die antivirale Therapie in Menschen, Tieren und Pflanzen.

16. Verwendung nach Anspruch 15 zur Herstellung eines Arzneimittels gegen AIDS.

17. Diagnostisches Reagenz,
**dadurch gekennzeichnet,**
daß es als Bestandteil eine Oligonukleotidstruktur nach einem der Ansprüche 1 bis 8 enthält.

18. Verfahren zur Herstellung eines diagnostischen Reagenz für eine genetische Musterungsprozedur,
**dadurch gekennzeichnet,**
daß es als Bestandteil eine Oligonukleotidstruktur nach einem der Ansprüche 1 bis 8 enthält.

## Claims

1. Synthetic catalytic oligonucleotide structure which is suitable for cleaving a nucleic acid target sequence and which contains nucleotides of the general structural formula (I): 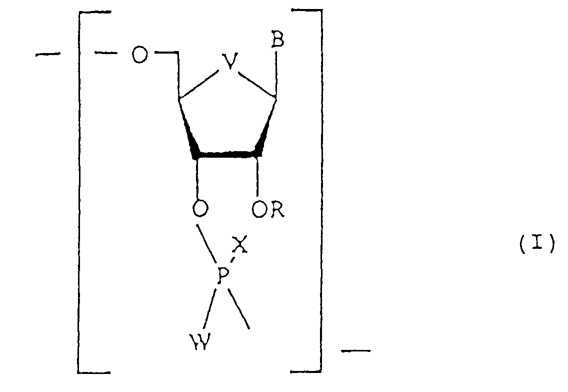 wherein B represents a nucleoside base which is in particular chosen from the group consisting of adenine-9-yl (A), cytosine-1-yl (C), guanine-9-yl (G), uracil-1-yl (U), uracil-5-yl (ψ), hypoxanthine-9-yl (I), thymine-1-yl (T) and 2-amino-adenine-9-yl,
V is in every nucleotide independently an O- or a CH₂-group,
X and W in a nucleotide can each be the same or different and independently from one another are O-, S-, NH₂-, alkyl- or alkoxy-groups with 1 to 10, preferably with 1 to 4 carbon atoms,
R is hydrogen or a straight chain or branched alkyl-, alkenyl-or alkynyl-group with 1 to 10 carbon atoms which is possibly substituted with halogen-, cyano-, isocyano-, nitro-, amino-, carboxyl-, hydroxyl- or/and mercapto-groups, characterized in that
in at least one of the nucleotides the moiety R in Formula (I) is different than hydrogen,
in the catalytically active center nucleotides exist in which R is hydrogen,
the catalytically active center of the oligonucleotide structure exhibits a hammerhead or a hairpin structure and
the oligonucleotide structure contains regions which are responsible for the hybridization with the nucleic acid target sequence.

2. Oligonucleotide structure according to claim 1, characterized in that the moieties V, W and X in Formula (I) are O-groups.

3. Oligonucleotide structure according to claim 1 or 2, characterized in that the moieties R in Formula (I) which are different than hydrogen contain 1 to 6 carbon atoms.

4. Oligonucleotide structure according to claim 3, characterized in that the moieties R in Formula (I) which are different than hydrogen are chosen from the group consisting of methyl, ethyl, propyl, allyl, dimethylallyl, butyl or cyanomethyl moieties.

5. Oligonucleotide structure according to one of the previous claims, characterized in that 3'-deoxyribonucleotides or/and 3'-0-alkylribonucleotides are located at the free 3'-ends of the oligonucleotide structure.

6. Oligonucleotide structure according to one of the previous claims, characterized in that it further contains nucleotides for the stabilization of its spatial configuration, the nucleoside bases of which are modified by a crosslinking agent.

7. Oligonucleotide structure according to claim 6, characterized in that the crosslinking agent is psoralen or a psoralen derivative.

8. Oligonucleotide structure according to one of the previous claims, characterized in that it is linked to a prosthetic group chosen from polyamino acids, lipids, hormones or peptides in order to improve uptake in the cell or/and the specific cellular localization of the oligonucleotide structure.

9. Method for cleaving a nucleic acid target sequence using one of the synthetic catalytic nucleotide structures according to one of the claims 1 to 10.

10. Method according to claim 9, characterized in that the nucleic acid targets sequence is a ribonucleic acid.

11. Method according to claim 9 or 10, characterized in that one carries out the cleavage in the presence of a divalent cation, particularly Mg²⁺.

12. Method according to one of the claims 9 to 11, characterized in that one carries out the cleavage at a pH of about 7 to 9.

13. Use an oligonucleotide structure according to one of the claims 1 to 8 for the catalytic cleavage of a nucleic acid target sequence.

14. Medicament, characterized in that it contains as its active ingredient an oligonucleotide structure according to one of the claims 1 to 8, possibly together with common pharmaceutical carrier, helping, filling or/and dilution agents.

15. Use of an oligonucleotide structure according to one of the claims 1 to 8 for producing a medicament for antiviral therapy in humans, animals and plants.

16. Use according to claim 15 for the production of a medicament against AIDS.

17. Diagnostic reagent, characterized in that it contains an oligonucleotide structure according to one of the claims 1 to 8 as an ingredient.

18. Method for producing a diagnostic reagent for a genetic screening procedure, characterized in that it contains an oligonucleotide structure according to one of the claims 1 to 8 as an ingredient.

## Revendications

1. Structure oligonucléotidique catalytique synthétique, qui convient pour la coupure d'une séquence cible d'acide nucléique, et qui renferme un nucléotide de formule développée générale (I) : 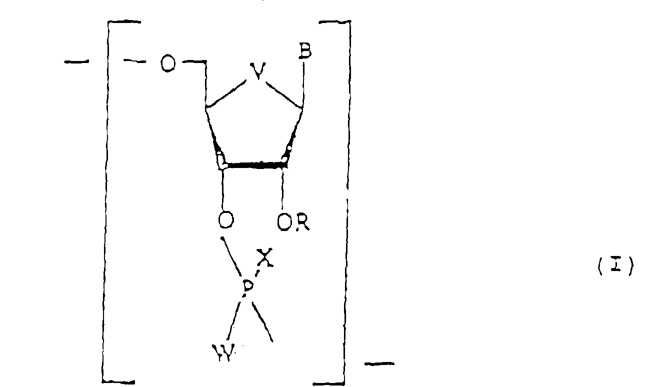 dans laquelle B représente une base nucléosidique, qui, en particulier, est choisie dans le groupe constitué de l'adénin-9-yle (A), de la cytosin-1-yle (C), de la guanin-9-yle (G), de l'uracyl-1-yle (U), de l'uracyl-5-yle (ψ) (I) de l'hypoxanthine-9-yle (1), de la thymin-1-yle (T) et de la 2-amino-adénin-9-yle,
V indépendamment de chaque nucléotide, est un O- ou bien un groupe CH₂,
X et W peuvent être identiques ou différents dans chaque nucléotide et, indépendamment l'un de l'autre, sont O-, S-, NH₂- ou des groupes alkyle ou alcoxy comportant 1 à 10, et de préférence 1 à 4 atomes de carbone,
R est un hydrogène ou un groupe alkyle, alkényle ou alkynyle, comportant 1 à 10 atomes de carbone, linéaire ou ramifié, éventuellement substitué par un halogène cyano, isocyano, nitro, amino, carboxyle, hydroxyle et/ou mercapto,
caractérisé en ce que,
dans au moins un des nucléotides, le reste R de la formule (I) n'est pas un hydrogène, en ce qu'il existe des nucléotides du centre catalytiquement actifs dans lesquels R est un hydrogène, en ce que le centre catalytiquement actif de la structure oligonucléotidique présente une structure en tête de marteau ou en épingle à cheveux et en ce que la structure oligonucléotidique renferme un domaine qui est responsable de l'hybridation avec la séquence cible de l'acide nucléique.

2. Structure oligonucléotidique selon la revendication 1, caractérisée en ce que les restes V, W et X de la formule (I) sont des groupes O.

3. Structure oligonucléotidique selon la revendication 1 ou 2, caractérisée en ce que le reste R autre qu'un hydrogène de la formule (I) renferme 1 à 6 atomes de carbone.

4. Structure oligonucléotidique selon la revendication 3, caractérisée en ce que le reste R autre que l'hydrogène de la formule (I) est choisi dans le groupe consistant en les restes méthyle, éthyle, propyle, allyle, diméthylallyle, butyle ou cyanométhyle.

5. Structure oligonucléotidique selon l'une quelconque des revendications précédentes, caractérisée en ce qu'à l'extrémité 3' libre de la structure oligonucléotidique se trouve un 3'-désoxyribonucléotide et/ou un 3'-O-alkylribonucléotide.

6. Structure oligonucléotidique selon l'une quelconque des revendications précédentes, caractérisée en ce que, en outre, pour la stabilisation de sa configuration spatiale, elle renferme des nucléotides dont les bases nucléotidiques sont modifiées par l'intermédiaire d'un agent de réticulation transversal.

7. Structure oligonucléotidique selon la revendication 6, caractérisée en ce que l'agent de réticulation transversal est un psoralène ou un dérivé de psoralène.

8. Structure oligonucléotidique selon l'une quelconque des revendications précédentes, caractérisée en ce qu'elle est liée à un groupement prosthétique, choisi parmi les acides polyaminés, les lipides, les hormones ou les peptides, afin d'améliorer l'absorption dans la cellule et/ou la location cellulaire spécifique de la structure oligonucléotidique.

9. Procédé de coupure d'une séquence cible d'acide nucléique en utilisant une structure oligonucléotidique catalytique synthétique selon l'une quelconque des revendications 1 à 10.

10. Procédé selon la revendication 9, caractérisé en ce que la séquence cible de l'acide nucléique est un acide ribonucléique.

11. Procédé selon la revendication 9 ou 10, caractérisé en ce que l'on effectue la coupure en présence de cations divalents, en particulier de Mg²⁺.

12. Procédé selon l'une quelconque des revendications 9 à 11, caractérisé en ce que l'on effectue la coupure à un pH de 7 à 9 environ.

13. Utilisation d'une structure oligonucléotique selon l'une quelconque des revendications 1 à 8 pour la coupure catalytique d'une séquence cible d'acide nucléique.

14. Médicament caractérisé en ce qu'il renferme en tant qu'agent actif, une structure oligonucléotidique selon une quelconque des revendications 1 à 8, éventuellement en association avec les agents supports, adjuvants, charges et/ou diluants pharmaceutiquement habituels.

15. Utilisation d'une structure oligonucléotidique selon une quelconque des revendications 1 à 8 pour la fabrication d'un médicament pour la thérapie antivirale chez l'homme, l'animal et les plantes.

16. Utilisation selon la revendication 15 pour la fabrication d'un médicament contre le SIDA.

17. Réactif de diagnostic caractérisé en ce qu'il contient en tant que constituant une structure oligonucléotidique selon l'une quelconque des revendications 1 à 8.

18. Procédé de fabrication d'un réactif de diagnostic pour un protocole d'établissement de carte génétique, caractérisé en ce qu'il renferme en tant que constituant une structure oligonucléotidique selon l'une quelconque des revendications 1 à 8.
